# EUROPEAN PATENT APPLICATION

(11) **EP 1 760 063 A1**
(43) Date of publication of application: **07.03.2007**
(21) Application number: 05734694.2
(22) Date of filing: 21.04.2005
(51) Int. Cl.: C07C 43/17, C07C 41/01

(54) **FLUORINE-CONTAINING VINYL ETHER COMPOUND AND PROCESS FOR PRODUCING THE SAME**

(30) Priority: 21.06.2004 JP 2004182129
(71) Applicant: Unimatec Co., Ltd., Tokyo 105-8585 (JP)
(72) Inventor: KOKIN, Keisuke, UNIMATEC CO., LTD., Kitaibaraki-shi, Ibaraki 319-1544 (JP); SONOI, Takehiro, UNIMATEC CO., LTD., Kitaibaraki-shi, Ibaraki 319-1544 (JP)
(74) Representative: Albrecht, Thomas
(86) International application number: PCT/JP2005/007614
(87) International publication number: WO 2005/123643

(57) **Abstract**

A fluorine-containing vinyl ether compound, represented by the following general formula :

RfO(C₃F₆O)ₐ(CₙF₂ₙ)(CₘH₂ₘ)_{b}CH₂OCH=CH₂

(where Rf is a polyfluoroalkyl group having 1 or more carbon atoms, a is 0, or an integer of 1-30, b is an integer of 1-10, and n and m each are 1 or 2), is produced by subjecting a fluorine-containing alcohol, represented by the following general formula :

RfO(C₃F₆O)ₐ(CₙF₂ₙ)(CₘH₂ₘ)_{b}CH₂OH

and an alkyl vinyl ether, represented by the following general formula :

XCH₂CH₂OCH=CH₂

(where X is a halogen atom, a hydrogen atom, or an alkyl group having 1-10 carbon atoms) to XCH₂CH₂OH-elimination reaction in the presence of a palladium-based catalyst, and has a fluorine-containing polyether group originating from the hexafluoropropene oxide polymer, and thus is not so rigid as the perfluoroalkyl alkyl group of perfluoroalkyl vinyl ether, rather more flexible, and furthermore has distinguished thermal and chemical stabilities, and also good optical properties and surface-active characteristics together.

## Description

### TECHNICAL FIELD

The present invention relates to a fluorine-containing vinyl ether compound and a process for producing the same, and more particularly to a novel fluorine-containing vinyl ether compound having a fluorine-containing polyether group and a process for producing the same.

### BACKGROUND ART

A process for producing fluoroalkyl vinyl ether by reaction of a fluoroalkyl alcohol with an alkyl vinyl ether in the presence of a palladium catalyst has been reported, where not only much excess alkyl vinyl ether is used relative to the fluoroalkyl alcohol, but also required reaction time is 72 hours or more and the yield is as small as about 75%, and when the crude product obtained by the reaction with a low percent conversion is purified by distillation, the distillation product is entrained with highly volatile fluoroalkyl alcohol, resulting in inefficient distillation as a problem :

Rf(CH₂)ₙOH+H(CH₂)ₘOCH=CH₂ → Rf(CH₂)ₙOCH=CH₂

n:2-6, m:1-6
Patent Literature 1 : WO92/05135
Non-Patent Literature 1 : Macromol. Chem., Vol.193, pp275-284(1992)

Another example of using similar reactive materials and mercury acetate Hg(OAc)₂ as a catalyst has been also reported, where not only the yield is as low as about 50%, but also use of a mercury-based catalyst is not preferable from the viewpoint of environmental burden.

C₆F₁₃CH₂CH₂OH+C₂H₅OCH=CH₂ → C₆F₁₃CH₂CH₂OCH=CH₂

Non-Patent Literature 2 : J. Fluorine Chem., Vol. 44, pp395-412(1989)

Furthermore, a process for synthesizing a desired alkyl vinyl ether R¹OCH=CH₂ by reaction of a hydrocarbon-based alcohol R¹OH (R¹ : an alkyl group having 10-18 carbon atoms) with an alkyl vinyl ether R²OCH=CH₂ (R² : an alkyl group having 1-4 carbon atoms) in the presence of a palladium catalyst has been also proposed, where the yield is increased by utilizing the nature of equilibrium reaction, that is, by distilling off the by-product alcohol R²OH originating from the raw material alkyl vinyl ether, formed as a result of reaction to the outside of the reaction system, thereby shifting the equilibrium.
Patent Literature 2 : JP-A-2001-114718

In the foregoing reaction, however, to shift the equilibrium towards the product side the boiling point of the by-product alcohol to be removed to the outside of the system must be the lowest among the raw material alcohol, raw material vinyl ether, product vinyl ether and by-product alcohol present in the reaction system, and consequently not only the structure of the product vinyl ether, but also those of the raw material alcohol and the raw material vinyl ether are considerably limited, so the versatility is not satisfactory. Furthermore, the necessity for discharging the by-product alcohol to the outside of the reaction system will complicate the reactor structure or experimental procedures, so the scale-up is not easy to conduct.

Still furthermore, a process for reaction of an alcohol ROH with vinyl acetate CH₃COOCH=CH₂ in the presence of an iridium catalyst [Ir(cod)Cl]₂ to synthesize the corresponding alkyl vinyl ether ROCH=CH₂ has been also reported, where the iridium catalyst is not only expensive, but the yield based on triethylene glycol as an alcohol is as low as 63%, it seems that the yield is largely influenced with the acidity of the alcohols.
Non Patent Literature 3 : J. Am. Chem. Soc., Vol. 124, pp1590-1591 (2002)

Besides the foregoing facts, all the examples of the foregoing reactions relate only to cases of converting aliphatic fluorine-containing alkyl alcohols to vinyl ether derivatives, and no examples have been reported about conversion of alcohols, etc., originating from a fluorine-containing hydroxypolyether, more specifically hexafluoropropene oxide polymer, to vinyl ether derivatives.

### DISCLOSURE OF THE INVENTION

### PROBLEM TO BE SOLVED BY THE INVENTION

An object of the present invention is to provide a fluorine-containing vinyl ether compound having a fluorine-containing polyether group originating from hexafluoropropene oxide polymer, that is, not so rigid as the perfluoroalkyl group of perfluoroalkyl vinyl ether, rather more flexible, and further having distinguished thermal and chemical stabilities, and also good optical properties and surface-active characteristics together.

### MEANS FOR SOLVING THE PROBLEM

The object of the present invention can be attained by a fluorine-containing vinyl ether compound, represented by the following general formula :

RfO(C₃F₆O)ₐ(CₙF₂ₙ)(CₘH₂ₘ)_{b}CH₂OCH=CH₂ [I]

(where Rf is a polyfluoroalkyl group having 1 or more carbon atoms, a is 0 or an integer of 1-30, b is an integer of 1-10, and n and m each are 1 or 2). The fluorine-containing vinyl ether compound can be produced by subjecting a fluorine-containing alcohol, represented by the following general formula :

RfO(C₃F₆O)ₐ(CₙF₂ₙ)(CₘH₂ₘ)_{b}CH₂OH [II]

(where Rf is a polyfluoroalkyl group having 1 or more carbon atoms, a is 0 or an integer of 1-30, b is an integer of 1-10, and n and m each are 1 or 2), and an alkyl vinyl ether, represented by the following general formula :

XCH₂CH₂OCH= CH₂ [III]

(where X is a halogen atom, a hydrogen atom, or an alkyl group having 1-10 carbon atoms) to XCH₂CH₂OH-elimination reaction in the presence of a palladium catalyst.

### EFFECT OF THE INVENTION

The present fluorine-containing vinyl ether compound can be obtained by XCH₂CH₂OH-elimination reaction of a fluorine-containing alcohol, represented by the general formula [II] and an alkyl vinyl ether, represented by the general formula [III], and particularly in the case of 2-haloethanol-elimination reaction, the desired product can be obtained in a percent conversion of 90% or more.

The fluorine-containing vinyl ether compound has the polymerization site on the hydrocarbon vinyloxy group, and thus the polymerization characteristics are better than those of perfluoroalkyl vinyl ether compounds, so it is used as a cross-linking agent or a modifying agent for various resins to improve or ameliorate various characteristics of resins, such as heat resistance, weathering resistance, chemical resistance, etc..

Due to the presence of a large number of ether bonds in the molecule, the compound is non-crystalline and highly flexible, and due to the higher fluorine content the compound has a low refractive index, and thus can be used as an anti-reflection film for display, etc., or as a clad material for optical fibers, etc. or furthermore due to the surface-active characteristics, it can be used as various surface coating agents, various mold-release coating agents, or a component for forming a surface-modifying agent and a water- and oil-repellent.

### BEST MODES FOR CARRYING OUT THE INVENTION

The fluorine-containing vinyl ether compounds, represented by the general formula [I] includes the following compounds, where the polyfluoroalkyl group Rf is the ones having 1 or more carbon atoms, usually 1-11 carbon atoms, preferably perfluoroalkyl groups, a is 0 or an integer of 1 or more, preferably 1-30, b is an integer of 1-10, preferably 1-4, and n and m each are 1 or 2, and preferably a -CF(CF3)- group of n=2 is used :

C₃F₇OCF(CF₃)CH₂CH₂OCH= CH₂

C₃F₇OCF(CF₃)CH₂CH₂CH₂OCH= CH₂

C₃F₇OCF(CF₃)CH₂CH₂CH₂CH₂OCH= CH₂

C₃F₇OCF(CF₃)CF₂OCF(CF₃)CH₂CH₂OCH= CH₂

C₃F₇OCF(CF₃)CF₂OCF(CF₃)CH₂CH₂CH₂OCH= CH₂

C₃F₇OCF(CF₃)CF₂OCF(CF₃)CH₂CH₂CH₂CH₂OCH= CH₂

C₃F₇O[CF(CF₃)CF₂O]₂CF(CF₃)CH₂CH₂OCH= CH₂

C₃F₇O[CF(CF₃)CF₂O]₂CF(CF₃)CH₂CH₂CH₂OCH= CH₂

C₃F₇O[CF(CF₃)CF₂O]₂CF(CF₃)CH₂CH₂CH₂CH₂OCH= CH₂

C₃F₇OCF₂CF₂CF₂OCF₂CF₂CH₂CH₂OCH= CH₂

C₃F₇OCF₂CF₂CF₂OCF₂CF₂CH₂CH₂CH₂OCH= CH₂

C₃F₇OCF₂CF₂CF₂OCF₂CF₂CH₂CH₂CH₂CH₂OCH= CH₂

C₃F₇O(CF₂CF₂CF₂O)₂CF₂CF₂CH₂CH₂OCH= CH₂

C₃F₇O(CF₂CF₂CF₂O)₂CF₂CF₂CH₂CH₂CH₂OCH= CH₂

C₃F₇O(CF₂CF₂CF₂O)₂CF₂CF₂CH₂CH₂CH₂CH₂OCH= CH₂

The fluorine-containing vinyl ether compound [I] can be produced by XCH₂CH₂OH-elimination reaction of a fluorine-containing alcohol, represented by the general formula [II], with an alkyl vinyl ether, represented by the general formula [III], in the presence of a palladium-based catalyst.

The fluorine-containing alcohol, represented by the general formula [II], can be obtained, when the C₃F₆O group is a -CF(CF₃)CF₂O-group, by subjecting hexafluoropropene oxide to anionic polymerization in the presence of a cesium fluoride catalyst, and converting the resulting terminal -CF(CF₃)COF- group to a -CF(CF₃)I- group by cesium carbonate and iodine, further to a -CF(CF₃)CH₂CH₂I- by ethylene addition, and finally to a -CF(CF₃)CH₂CH₂OH group by hydrolysis. The same reaction is also applicable to the case of a=0, corresponding to hexafluoropropene oxide dimer.
Non-Patent Literature 4 : J. Fluorine Chemistry, Vol. 65, pp. 59-65(1993)
Patent Literature 3 : JP No. 2,603,171

When the C₃F₆O group is a -CF₂CF₂CF₂O- group the desired compound can be obtained by subjecting 2,2,3,3-tetrafluorooxetane to anionic polymerization in the presence of a cesium fluoride catalyst, conducting a fluorine gas treatment of the resulting fluorine-containing polyether (CH₂CF₂CF₂O)ₙ to obtain (CF₂CF₂CF₂O)ₙ, followed by the same treatment as in the afore-mentioned case of the -CF(CF₃)COF- group, thereby conducting conversion to a -CF₂CF₂CF₂OCF₂CH₂CH₂OH- group.

The alkyl vinyl ether to react with the fluorine-containing alcohol is preferably 2-haloetheyl vinyl ether, where X is a halogen atom, preferably a chlorine atom or a bromine atom. When butyl vinyl ether, where X is an ethyl group, is used, the percent conversion is about 60 to about 70% at best, whereas with 2-haloethyl vinyl ether the percent conversion can reach 90% or more. This is because the XCH₂CH₂OH- elimination reaction is a reversible reaction, and the percent conversion can be increased and the reverse reaction can be suppressed with 2-haloethyl vinyl ether having a distinguished releasability. Thus, it seems that the percent conversion can be remarkably increased. The alkyl vinyl ether can be used in excess moles, particularly preferably in a proportion of about 10 parts by mole per mole of the fluorine-containing alcohol.

The XCH₂CH₂OH-elimination reaction between the fluorine-containing alcohol and the alkyl vinyl ether is carried out in the presence of a palladium-based catalyst. The palladium-based catalyst for use herein includes palladium (1,10-phenanthroline) acetate, palladium (2,2'-bipyridyl) acetate, palladium bis(triphenylphosphino) acetate, etc. Particularly preferable are palladium (1,10-phenanthroline) acetate and palladium (2,2'-bipyridyl) acetate. The catalyst can be used in a proportion of 0.5% by mole or more, particularly preferably 0.5-2.5% by mole, on the basis of the fluorine-containing alcohol.

The reaction can be carried out without any solvent, but a solvent can be used. That is, an appropriate amount of a solvent selected from hydrocarbon-based solvents such as toluene, xylene, etc., and hydrocarbon cyclic ether-based solvents such as tetrahydrofuran, 1,4-dioxane, etc. can be used.

To suppress polymerization of fluorine-containing vinyl ether resulting from the reaction, it is effective to add an alkali metal hydroxide to the reaction system. The alkali metal hydroxide such as sodium hydroxide, potassium hydroxide, etc. can be used in a proportion of about 0.01-5% by weight, particularly preferably 0.05-1% by weight, on the basis of the fluorine-containing alcohol.

The reaction temperature is not particularly limited, and is usually room temperature to 80°C, particularly preferably 40 ° -60°C. When the reaction is carried out particularly above room temperature, the effect of heating is very remarkable, and the necessary reaction time of 48 hours at room temperature can be largely shortened, so the same percent conversion and yield can be obtained within 8 hours.

Unreacted alkyl vinyl ether, by-product alcohols, and product fluorine-containing vinyl ether compound can be purified by fractional distillation, and those having larger molecular weights and higher boiling points can be purified by molecular distillation, and can be used to reactions, such as polymerization, etc.

### EXAMPLES

The present invention will be described in detail below, referring to Examples.

### EXAMPLE 1

20g (30.2millimoles) of 2-(perfluoro-3,6,9-trioxo-5,8-dimethyldodeca-2-yl)ethane-1-ol (purity : 98.9GC%), 32.2g (302millimoles) of 2-chloroethyl vinyl ether, and 0.28g (692nanomoles) of palladium (1,10-phenanthroline) acetate were added to a flask having a net capacity of 100ml, provided with a reflux condenser, a thermometer, and a stirrer in a nitrogen gas atmosphere, and heated with stirring for 5 hours to keep the internal temperature at 50° -55°C.

The palladium (1,10-phenanthroline) acetate was synthesized as 4.6g of yellow crystals (yield : 85.6%) by adding 300ml of a toluene solution containing 2.4g (13.4millimoles) of 1,10-phenanthroline to 300ml of a toluene solution containing 3g (13.4millimoles) of palladium acetate, stirring the mixture at room temperature, recovering crystals from the reaction solution with changing color from dark brown to orange by filtration, followed by drying under reduced pressure.

The reaction mixture obtained by heating with stirring for 5 hours was separated into two layers, which were isolated as an upper layer and a lower layer, respectively. Determination by ¹⁹F-NMR, ¹H-NMR, and gas chromatography (GC) revealed that the lower layer was the desired 2-(perfluoro-3,6,9-trioxo-5,8-dimethyldodeca-2-yl) ethyl vinyl ether (purity : 72.8GC%, yield : 92.3%).

C₃F₇OCF(CF₃)CF₂OCF(CF₃)CF₂OCF(CF₃)CH₂CH₂OCH= CH₂

¹⁹F-NMR (acetone-d6, CFCl₃):
- 77.86~-80.67(m, CFCF₂O, CF₂O, 6F)
- 79.03(s, CF₃CFO, 6F)
- 80.49(s, CF₃CF₂, 3F)
- 78.75(dbr, CF₃CFCH₂, 3F)
- 126.37(ddt, CFCH₂**,** 1F, J_{HF}=169Hz)
- 128.61(s, CF₃CF₂, 2F)
- 143.64~-144.15(m, CF(CF₃)CF₂O, 2F)
¹H-NMR (acetone-d6, TMS):
6.51(dd, OHC=C, 1H, J_{HH}=6.79Hz, J_{HH}=14.3Hz)
4.24(dbr, C=CH₂, 1H, J_{HH}=14.3Hz)
4.07~3.97(m, CH₂CH₂O, 2H)
3.80(dbr, C=CH₂, 1H, J_{HH}=6.59Hz)
2.78(dt, CFCH₂, 2H, J_{HH}=17.4Hz, J_{HH}=6.6Hz)

### EXAMPLE 2

10g (15.5millimoles) of 3-(perfluoro-3,6,9-trioxo-5,8-dimethydodeca-2-yl)propane-1-ol (purity : 98.9GC%), 15.8g (148millimole) of 2-chloroethyl vinyl ether, 0.13g (312nanomoles) of palladium (1,10-phenanthroline) acetate, and 0.01g of potassium hydroxide were added to a flask having a net capacity of 100ml, provided with a reflux condenser, a thermometer, and a stirrer under a nitrogen gas atmosphere, and heated with stirring for 5 hours to keep the internal temperature at 50° -55°C.

The resulting reaction mixture was separated into two layers, which were isolated as an upper layer and a lower layer, respectively. Determination by ¹⁹F-NMR, ¹H-NMR, and GC revealed that the lower layer was the desired 3-(perfluoro-3,6,9-trioxo-5,8-dimethyldodeca-2-yl)propyl vinyl ether (purity : 85.3GC%, yield : 88.0%).

C₃F₇OCF(CF₃)CF₂OCF(CF₃)CF₂OCF(CF₃)CH₂CH₂CH₂OCH= CH₂

¹⁹F-NMR (acetone-d6, CFCl₃) :
- 77.76~-80.58(m, CFCF₂O, CF₂O, 6F)
- 79.02(s, CF₃CFO, 6F)
- 80.46(s, CF₃CFCH₂, 3F)
- 82.17(dd, CF₃CF₂, 3F, J_{FF}=39.3Hz, J_{FF}=8.48Hz)
- 126.63(ddt, CFCH₂, 1F, J_{HF}=119Hz)
- 128.60(s, CF₃CF₂, 2F)
- 143.64~-144.18(m, CF(CF₃)CF₂O, 2F)
¹H-NMR (acetone-d6, TMS) :
6.50(dd, OHC=C, 1H, J_{HH}=6.79Hz, J_{HH}=14.3Hz)
4.22(dd, C=CH₂, 1H, J_{HH}=14.4Hz)
3.98(t, CH₂CH₂O, 2H, J_{HH}=3.30Hz)
3.80(dbr, C=CH₂, 1H, J_{HH}=6.59Hz)
2.48(dt, CF₃CFCH₂, 2H, J_{HF}=16.6Hz, J_{HH}=7.9Hz)
1.95(tt, CH₂CH₂CH₂, 2H, J_{HH}=16.3Hz)

### EXAMPLE 3

20g (30.2millimoles) of 2-(perfluoro-3,6,9-trioxo-5,8-dimethyldodeca-2-yl)ethane-1-ol (purity : 98.9GC%), 32.2g (302millimoles) of 2-chloroethyl vinyl ether, and 0.28g (692nanomoles) of palladium (1,10-phenanthroline) acetate were added to a flask having a net capacity of 100ml, provided with a reflux condenser, a thermometer, and a stirrer under a nitrogen gas atmosphere, and heated with stirring at room temperature for 48 hours.

The resulting reaction mixture was separated into two layers, which were isolated as an upper layer and a lower layer, respectively. Determination by ¹⁹F-NMR, ¹H-NMR, and GC revealed that the lower layer was the desired 2-(perfhioro-3,6,9-trioxo-5,8-dimethyldodeca-2-yl)ethyl vinyl ether (purity : 65.4GC%, yield: 68.0%).

### EXAMPLE 4

20g (30.2millimole) of 2-(perfluoro-3,6,9-trioxo-5,8-dimethyldodeca-2-yl)ethane-1-ol(purity : 98.9GC%), 30.2g (302millimoles) of butyl vinyl ether, 0.28g (692nanomoles) of palladium (1,10-phenanthroline) acetate, and 0.01g of potassium hydroxide were added to a flask having a net capacity of 100ml, provided with a reflux condenser, a thermometer, and a stirrer under a nitrogen gas atmosphere, and heated with stirring for 5 hours to keep the internal temperature at 50° -55°C.

The resulting reaction mixture was separated into two layers, which were isolated as an upper layer and a lower layer, respectively. Determination by ¹⁹F-NMR, ¹H-NMR, and GC revealed that the lower layer was the desired 2-(perfluoro-3,6,9-trioxo-5,8-dimethyldodeca-2-yl)ethyl vinyl ether (purity : 58GC%, yield : 62.0%).

C₃F₇OCF(CF₃)CF₂OCF(CF₃)CF₂OCF(CF₃)CH₂CH₂OCH= CH₂

## Claims

1. A fluorine-containing vinyl ether compound, represented by the following general formula :
RfO(C₃F₆O)ₐ(CₙF₂ₙ)(CₘH₂ₘ)_{b}CH₂OCH=CH₂ [I]
where Rf is a polyfluoroalkyl group having 1 or more carbon atoms, a is 0, or an integer of 1-30, b is an integer of 1-10, and n and m each are 1 or 2.

2. A process for producing a fluorine-containing vinyl ether compound, represented by the following general formula :
RfO(C₃F₆O)ₐ(CₙF₂ₙ)(CₘH₂ₘ)_{b}CH₂OCH=CH₂ [I]
where Rf, a, b, c, n and m have the same meanings as defined below, **characterized by** subjecting a fluorine-containing alcohol, represented by the following general formula :
RfO(C₃F₆O)ₐ(CₙF₂ₙ)(CₘH₂ₘ)_{b}CH₂OH [II]
, where Rf is a polyfluoroalkyl group having 1 or more carbon atoms, a is 0 or an integer of 1-30, b is an integer of 1-10, and n and m each are 1 or 2, and an alkyl vinyl ether, represented by the following general formula :
XCH₂CH₂OCH=CH₂ [III]
where X is a halogen atom, a hydrogen atom, or an alkyl group having 1-10 carbon atoms, to XCH₂CH₂OH-elimination reaction in the presence of a palladium-based catalyst.

3. A process for producing a fluorine-containing vinyl ether compound according to Claim 2, wherein the palladium-based catalyst is palladium (1,10-phenanthroline) acetate, palladium (2,2'-bipyridyl) acetate, or palladium bis(triphenylphosphino) acetate.

4. A process for producing a fluorine-containing vinyl ether compound according to Claim 2, wherein the reaction is carried out in the presence of an alkali metal hydroxide as a polymerization inhibitor for the formed fluorine-containing vinyl ether compound.
